# EUROPEAN PATENT APPLICATION

(11) **EP 1 897 958 A2**
(43) Date of publication of application: **12.03.2008**
(21) Application number: 07115778.8
(22) Date of filing: 06.09.2007
(51) Int. Cl.: C12Q 1/68

(54) **Method, polynucleotide probe, DNA chip and kit for identifying mutation of human mitochondrial DNA**

(30) Priority: 07.09.2006 KR 20060086428
(71) Applicant: Genocheck Co., Ltd., Gyeonggi-do, 426-170 (KR)
(72) Inventor: Hwang, Seung Yong, 426-752, Ansan-si, Gyeonggi-do (KR); Yoon, Hyun Kyu, Seoul (KR)
(74) Representative: Behnisch, Werner

(57) **Abstract**

Disclosed are a method, a polynucleotide probe, a DNA chip and a kit for identifying a mutation of human mitochondrial DNA. The method comprises the steps of: isolating DNA from human blood, cells or tissues; subjecting the isolated DNA to polymerase chain reaction (PCR) to produce a PCR product; hybridizing the PCR product with a polynucleotide probe comprising a base sequence identical or complementary to one of DNA sequences of SEQ ID NOS.: 5 to 90; and examining hybridization between the PCR product and the polynucleotide probe to determine the mutation of the human mitochondrial DNA. Using this method, a plurality of mutations of human mtDNA and haplotypes of human mtDNA can be rapidly and accurately inspected on one slide.

## Description

### CROSS-REFERENCE TO RELATED PATENT APPLICATION

This application claims the benefit of Korean Patent Application No. 10-2006-0086428, filed on September 7, 2006, in the Korean Intellectual Property Office, the disclosure of which is incorporated herein in its entirety by reference.

### BACKGROUND OF THE INVENTION

### 1. Field of the Invention

The present invention relates to a method, polynucleotide probe, DNA chip and kit for identifying mutations of human mitochondrial DNA. Particularly, the present invention relates to a method for more simply, rapidly and accurately identifying mutations in human mitochondrial DNA by using DNA sample extracted human body. More particularly, the present invention relates to a polynucleotide probe based on single nucleotide polymorphism of the control region of the human mitochondrial DNA and a kit comprising the polynucleotide.

### 2. Description of the Related Art

The human mitochondrion is a cytoplasmic organelle having its own DNA, apart from a nucleus. The number of mitochondria present in a cell may range from tens to hundreds. Each mitochondrion contains up to 11 copies of its genome. The mitochondrial genome consists of 16,569 bp encoding 13 polypeptides, 22 tRNA genes and 2 rRNA genes. In mammals, mitochondria is inherited through the maternal lineage, with no recombination processes.

Therefore, mitochondrial DNA (mtDNA) is useful in the study of human evolution. Mitochondrial DNA is also used in forensic science as a tool for identifying individuals. In particular, mtDNA can be extracted from samples having little nuclear DNA, such as hair shafts, bones, teeth, etc., or samples which are too corrupt to subject to STR (short tandem repeat) analysis. There was an actual case in which mtDNA was isolated from a fossil of a Neanderthal man who lived and died 30000 years ago and was successfully analyzed to determine the base sequence. In most cases, the complete human mtDNA base sequence (CRS: Cambridge Reference Sequence) discovered by Anderson and his colleagues is used as a standard base sequence.

Mutation is the unique and most important factor causing the genetic alteration of mtDNA and is generated by substitution, deletion or insertion. Of them, substitution most frequently takes place. Over the long evolution period of time, specific loci on mtDNA have mutated at higher mutation rates than have other loci. The substitution rate of mitochondrial genomes is about 5-10 times higher than that of nuclear DNA. Particularly, the control region is inferred to evolve 5-20 times as fast as other regions. The control region, also called Dloop (Displacement loop), is about 1,100 bp long and comprises a replication origin and various regulatory factors. Most mtDNA mutations are focused on two hypervariable regions HV1 and HV2, each 400 bp long. Heteroplasmy is frequently found due to the high mutation rate of mtDNA. Therefore, because there are differences in the base sequence of mtDNA within the same maternal lineage group and even in the same individual, particular attention is paid to data analysis. With the growing spread of globalization over the globe, distinguishing between human races through mtDNA analysis is attracting increasing attention. Therefore, there exists a need for a simple and rapid methodology which allows a successful paternity test through the analysis of human genetic mutation and haplotype and maternal lineage on the basis of versatile base sequences detected in HV1 and HV2 of the mitochondrial DNA control region from specific human racial groups.

Thus far, the mutation of mtDNA has been typically analyzed by applying Sanger's sequencing method to the two hypervariable regions of the control region. Although it can secure accurate sequence numbers, the Sanger's method requires a large quantity of time for analysis data, and is inefficient because it is complex and based on many experimental processes. Other methods, for example, a minisequencing method and a sequence-specific probe method, are advantageous in that they are simple and can obtain results within a short period of time, but suffer from the disadvantage of having low analysis accuracy. In order to solve these problems, a methodology with which many mutations of human mtDNA can be identified in one experiment is needed.

### SUMMARY OF THE INVENTION

Accordingly, the present invention has been made keeping in mind the above problems occurring in the prior art, and an object of the present invention is to provide a method for identifying mutations of human mtDNA by isolating DNA from human blood, cells or tissues, amplifying the isolated DNA through polymerase chain reaction (PCR) to produce a PCR product, and hybridizing the PCR product with a polynucleotide probe comprising a base sequence identical or complementary to one of the DNA sequences of SEQ ID NOS.: 5 to 90 to examine human mtDNA variations, single nucleotide polymorphism (SNP), and haplotypes.

Although a number of differences in the base sequence of human mtDNA are disclosed, a lot of time and manpower is required to analyze the base sequences of human mtDNA of individual persons unless a standard methodology is established. In the present invention, accurate differences between DNA sequences of human mtDNA are detected and used as the basis on which polynucleotide probes capable of hybridizing with various base sequences corresponding to mutations of human mtDNA are constructed.

Also, a method is provided for rapidly and accurately analyzing the difference in base sequence of human mtDNA between individual persons using a DNA chip or a kit comprising the polynucleotide probe.

In addition, it is another object of the present invention to provide use of a polynucleotide probe, which is a consecutive nucleotide sequence 14 - 30 bp long containing a mutation site on human mitochondrial DNA, a DNA chip comprising the polynucleotide probe, and a kit comprising the DNA chip. Using the kit, a plurality of mutations of human mtDNA and haplotypes of human mtDNA can be rapidly and accurately inspected on one slide.

In accordance with an aspect of the present invention for accomplishing the above objects, there is provided a method of identifying a mutation of human mitochondrial DNA, comprising the steps of: isolating DNA from human blood, cells or tissues; subjecting the isolated DNA to polymerase chain reaction (PCR) to produce a PCR product; hybridizing the PCR product with a polynucleotide probe comprising a base sequence identical or complementary to one of DNA sequences of SEQ ID NOS.: 5 to 90; and examining the hybridization between the PCR product and the polynucleotide probe to determine mutations of the human mitochondrial DNA.

In accordance with another aspect of the present invention for accomplishing the above objects, there is provided a polynucleotide probe for identifying a mutation of human mitochondrial DNA, comprising a base sequence consisting of 14 ~ 30 consecutive nucleotide residues, which is identical or complementary to one of DNA sequences of SEQ ID NOS.: 5 to 90, whereby it can be used in the method of one of claims 1 to 6.

In accordance with a further aspect of the present invention, there is provided a DNA chip for identifying a mutation of human mitochondrial DNA, comprising a polynucleotide probe, which is a 14 - 30 consecutive bp long nucleotide sequence identical or complementary to one of the DNA sequences of SEQ ID NOS.: 5 to 90, and is capable of hybridizing with a base sequence containing an SNP locus on HV1 or HV2 of a control region of human mitochondrial DNA.

In accordance with still a further aspect of the present invention, there is provided a kit for identifying a mutation of human mitochondrial DNA, comprising: a polynucleotide probe, comprised of a consecutive nucleotide sequence 14 - 30 bp long identical or complementary to one of the DNA sequences of SEQ ID NOS.: 5 to 90, for hybridizing with a base sequence containing an SNP locus on HV1 or HV2 of a control region of human mitochondrial DNA; and a set of primers for amplifying human mitochondrial DNA through PCR.

### BREIF DESCRIPTION OF THE DRAWINGS

The above and other objects, features and other advantages of the present invention will be more clearly understood from the following detailed description taken in conjunction with the accompanying drawings, in which:
FIG. 1 is a schematic view showing a gene map of human mtDNA;
FIG. 2 is a base sequence information table showing SNP loci of polynucleotide probes in accordance with a preferable embodiment of the present invention;
FIG. 3 is an electophoresis photograph showing PCR products of the mtDNA isolated from humans;
FIG. 4 is a schematic diagram showing the structure of a DNA chip containing polynucleotide probes according to a preferable embodiment of the present invention;
FIG. 5 is a schematic diagram showing the constitution of a DNA chip in which polynucleotide probes responsible for the HV2 gene are arranged; and
FIGS. 6A to 6C are photographs showing results from the hybridization of PCR products of human mtDNA with the polynucleotide probes of the present invention.

### DESCRIPTION OF THE PREFERRED EMBODIMENTS

With reference to the accompanying drawings, a detailed description will be given of the present invention below.

In accordance with an aspect thereof, the present invention provides a method of identifying the mutation of human mtDNA. Human mtDNA may be isolated from human blood, cells or tissues. The sample is amplified through PCR.

Because the human mtDNA sample is used to identify mutations thereon, no particular limitations are imposed on the source and the isolation method thereof. Preferably, the isolated DNA sample contains the control region of human mtDNA.

In order to increase the amount of the sample isolated, PCR is performed. As long as it can multiply the mtDNA sample, any amplification technique may be employed. Thus, it should be noted that any DNA isolation or amplification methods known to those skilled in the art fall into the scope of the present invention. Particularly, the method of the present invention targets the base sequence of HV1 or HV2 on the control region of human mtDNA. PCR is preferably performed with a set of primers suitable for the amplification of HV1 and HV2 regions. Hence, the PCR product thus obtained preferably contains at least one of HV1 and HV2 of the control region of human mtDNA.

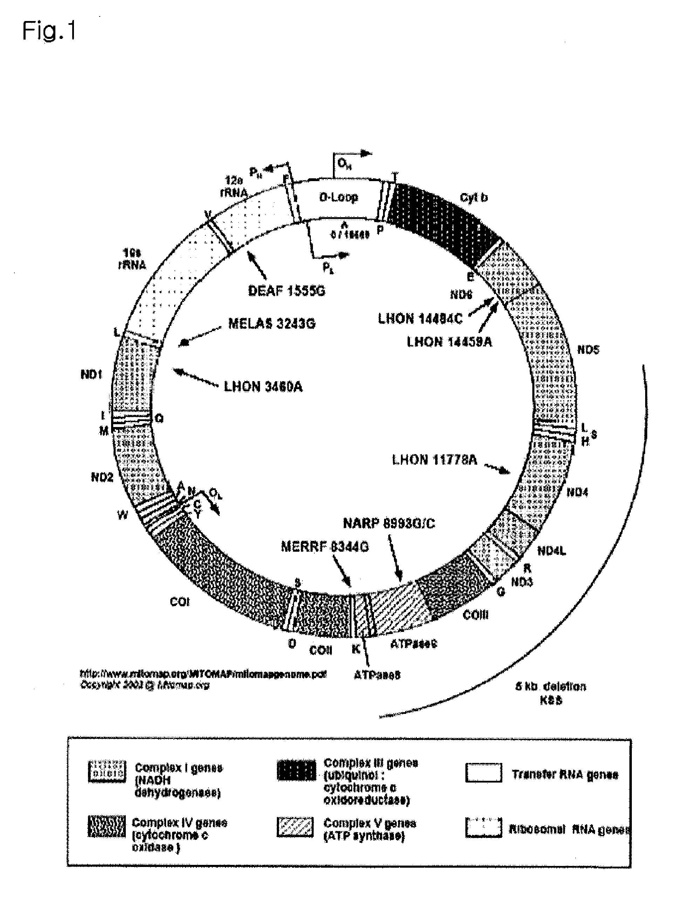

On the basis of the SNP sequences in HV1 and HV2 of the control region of human mtDNA, which were found by the present inventors, polynucleotide probes which can specifically bind to the SNP sequences are constructed. FIG. 1 is a schematic view showing a gene map of human mtDNA. As seen in this gene map, human mtDNA is 16,569 bp long and has a 1,100 bp long control region (also called "D-loop") with position 0 centered therein. In the control region, both HV1 and HV2 gene regions are located.

The most important feature of the method according to the present invention is to hybridize the PCR product with a polynucleotide probe which comprises a base sequence identical or complementary to one DNA sequence selected from a group consisting of SEQ ID NOS.: 5 to 90. Thereafter, hybridization is examined to determine mutation of the human mtDNA. In this regard, any technique that is generally known in the art may be used to examine whether hybridization between the PCR product and the probe has successfully occurred or not.

DNA sequences of SEQ ID NOS.: 5 to 90 according to the present invention are given in Table 1, below.

**TABLE 1**

| DNA Sequences for Use in Identifying Mutations of Human mtDNA | | | |
|---|---|---|---|
| DNA sequences for identifying mutations of human mtDNA | | | |
| Probes | DNA Sequences | Mutati on | Loci |
| 16066G (SEQ ID NO.:5) | 5'-CAA GTA TTG GCT CAC CCA TC -3' | A>G | 16066 (HV1) |
| 16075C (SEQ ID NO.:6) | 5'-CTC ACC CAC CAA CAA CC -3' | T>C | 16075 (HV1) |
| 16086C (SEQ ID NO.:7) | 5'-CAA CAA CCG CCA TGT ATT TCG -3' | T>C | 16086 (HV1) |
| 16093C (SEQ ID NO.:8) | 5'-CGC TAT GTA TCT CGT ACA TTA -3' | T>C | 16093 (HV1) |
| 16111T (SEQ ID NO.:9) | 5'-TTA CTG CCA GTC ACC ATG AAT -3' | C>T | 16111 (HV1) |
| 16124C (SEQ ID NO.: 10) | 5'-CCA TGA ATA TCG TAC GGT ACC -3' | T>C | 16124 (HV1) |
| 16126C (SEQ ID NO.: 11) | 5'-ATG AAT ATT GCA CGG TAC CA -3' | T>C | 16126 (HV1) |
| 16129A (SEQ ID NO.: 12) | 5'-ATG AAT ATT GTA CAG TAC CA -3' | G>A | 16129 (HV1) |
| 16126C 16129A (SEQ ID NO.: 13) | 5'-CCA TGA ATA TTG CAC AGT ACC ATA AA -3' | T>C, G>A | 16126,161 29 (HV1) |
| 16136C (SEQ ID NO.:14) | 5'-GTA CCA CAA ATA CTT GAC CA -3' | T>C | 16136 (HV1) |
| 16140C (SEQ ID NO.:15) | 5'-GTA CCA TAA ACA CTT GAC CA -3' | T>C | 16140 (HV1) |
| 16154T (SEQ ID NO.:16) | 5'-CTT GAC CAC CTG TAG TAC ATA A - 3' | | 16154 (HV1) |
| 16162G (SEQ ID NO.: 17) | 5'-CTG TAG TAC ATG AAA ACC CAA -3' | A>G | 16162 (HV1) |
| 16172C (SEQ ID NO.:18) | 5'-AAA ACC CAA CCC ACA TCA A -3' | T>C | 16172 (HV1) |
| 16174T (SEQ ID NO.: 19) | 5'-GTA CAT AAA AAC CCA ATC TAC ATC A -3' | C>T | 16174 (HV1) |
| 16192T (SEQ ID NO.:20) | 5'-TCA AAA CCC CCT CCT CAT GC -3' | C>T | 16192 (HV1) |
| 16209C (SEQ ID NO.:21) | 5'-TAC AAG CAA GCA CAG CAA TC -3' | T>C | 16209 (HV1) |
| 16213A (SEQ ID NO.:22) | 5'-CAA GCA AGT ACA ACA ATC AAC C -3' | G>A | 16213 (HV1) |
| 16217C (SEQ ID NO.:23) | 5'-GCA AGT ACA GCA ACC AAC C -3' | T>C | 16217 (HV1) |
| 16223T (SEQ ID NO.:24) | 5'-CAG CAA TCA ACC TTC AAC TAT CAC -3' | C>T | 16223 (HV1) |
| 16217C16223T (SEQ ID NO.:25) | 5'-GCA ACC AAC CCT CAA CTA -3' | T>C, C>T | 16217,162 23 (HV1) |
| 16224C (SEQ ID NO.:26) | 5'-AAT CAA CCC CCA ACT ATC A -3' | T>C | 16224 (HV1) |
| 16227G (SEQ ID NO.:27) | 5'-CAA CCC TCA GCT ATC ACA CA -3' | A>G | 16227 (HV1) |
| 16231C (SEQ ID NO.:28), | 5'-TCA ACT ACC ACA CAT CAA CTG -3' | T>C | 16231 (HV1) |
| 16232A (SEQ ID NO.:29) | 5'-CCC CTA ACT ATA ACA CAT CAA C - 3' | G>A | 16232 (HV1) |
| 16234T (SEQ ID NO.:30) | 5'-TCA ACT ATC ATA CAT CAA CTG -3' | C>T | 16234 (HV1) |
| 16235G (SEQ ID NO.:31) | 5'-CAA CTA TCA CGC ATC AAC TG -3' | A>G | 16235 (HV1) |
| 16243C (SEQ ID NO.:32) | 5'-ACA TCA ACC GCA ACT CC -3' | T>C | 16243 (HV1) |
| 16245T (SEQ ID NO.:33) | 5'-ACA TCA ACT GTA ACT CCA AAG -3' | C>T | 16245 (HV1) |
| 16249C (SEQ ID NO.:34) | 5'-CAT CAA CTG CAA CCC CAA AG -3' | T>C | 16249 (HV1) |
| 16256T (SEQ ID NO.:35) | 5'-CTC CAA AGT CAC CCC TC -3' | C>T | 16256 (HV1) |
| 16257A (SEQ ID NO.:36) | 5'-TGC AAC TCC AAA GCA ACC -3' | C>A | 16257 (HV1) |
| 16260T (SEQ ID NO.:37) | 5'-AAA GCC ACT CCT CAC CC -3' | C>T | 16260 (HV1) |
| 16261T (SEQ ID NO.:38) | 5'-AGC CAC CTC TCA CCC -3' | C>T | 16261 (HV1) |
| 16257A16261T (SEQ ID NO.:39) | 5'-CCA AAG CAA CCT CTC ACC -3' | C>A, C>T | 16257,162 61 (HV1) |
| 16265T (SEQ ID NO.:40) | 5'-ACC CCT CCC CCA CT -3' | A>T | 16265 (HV1) |
| 16266T (SEQ ID NO.:41) | 5'-ACC CCT CAT CCA CTA GG -3' | C>T | 16266 (HV1) |
| 16270T (SEQ ID NO.:42) | 5'-CCT CAC CCA TTA GGA TAC C -3' | C>T | 16270 (HV1) |
| 16274A (SEQ ID NO.:43) | 5'-CAC CCA CTA GAA TAC CAA CAA -3' | G>A | 16274 (HV1) |
| 16278T (SEQ ID NO.:44) | 5'-CAC TAG GAT ATC AAC AAA CCT -3' | C>T | 16278 (HV1) |
| 16264T16270T16278T (SEQ ID NO.:45) | 5'-CCC TTA CCC ATT AGG ATA TCA AC -3' | C>T, C>T, C>T | 16264,162 70,16278 (HV1) |
| 16284G (SEQ ID NO.:46) | 5'-ATA CCA ACA GAC CTA CCC A -3' | A>G | 16284 (HV1) |
| 16288C (SEQ ID NO.:47) | 5'-CAA CAA ACC CAC CCA CCC T -3' | T>C | 16288 (HV1) |
| 16290T (SEQ ID NO.:48) | 5'-AAC CTA TCC ACC CTT AAC AG -3' | C>T | 16290 (HV1) |
| 16291T (SEQ ID NO.:49) | 5'-AAC CTA CTC ACC CTT AAC AG -3' | C>T | 16291 (HV1) |
| 16293G (SEQ ID NO.:50) | 5'-AAC CTA CCC GCC CTT AAC -3' | A>G | 16293 (HV1) |
| 16294T (SEQ ID NO.:51) | 5'-AAC CTA CCC ATC CTT AAC AG -3' | C>T | 16294 (HV1) |
| 16295T (SEQ ID NO.:52) | 5'-AAC CTA CCC ACT CTT AAC AGT A - 3' | C>T | 16295 (HV1) |
| 16297C (SEQ ID NO.:53) | 5'-TAC CCA CCC CTA ACA GTA C -3' | T>C | 16297 (HV1) |
| 16298C (SEQ ID NO.:54) | 5'-ACC CAC CCT CAA CAG TAC AT -3' | T>C | 16298 (HV1) |
| 16304C (SEQ ID NO.:55) | 5'-TAA CAG CAC ATA GTA CAT AA -3' | T>C | 16304 (HV1) |
| 16309G (SEQ ID NO.:56) | 5'-CTT AAC AGT ACA TGG TAC AT -3' | A>G | 16309 (HV1) |
| 16311C (SEQ ID NO.:57) | 5'-CAG TAC ATA GCA CAT AAA GCC -3' | T>C | 16311 (HV1) |
| 16316G (SEQ ID NO.:58) | 5'-ATA GTA CAT GAA GCC ATT TAC -3' | A>G | 16316 (HV1) |
| 16319A (SEQ ID NO.:59) | 5'-AGT ACA TAA AAC CAT TTA CCG -3' | G>A | 16319 (HV1) |
| 16324C (SEQ ID NO.:60) | 5'-ATA AAG CCA TCT ACC GTA CAT -3' | T>C | 16324 (HV1) |
| 16325C (SEQ ID NO.:61) | 5'-ATA AAG CCA TTC ACC GTA CAT -3' | T>C | 16325 (HV1) |
| 16327T (SEQ ID NO.:62) | 5'-AAG CCA TTT ATC GTA CAT AGC -3' | C>T | 16327 (HV1) |
| 16355T (SEQ ID NO.:63) | 5'-GTC AAA TCC TTT CTC GCC C -3' | C>T | 16355 (HV1) |
| 16357C (SEQ ID NO.:64) | 5'-TCA AAT CCC TCC TCG TCC CCA -3' | T>C | 16357 (HV1) |
| 16362C (SEQ ID NO.:65) | 5'-CCT TCT CGC CCC CAT G -3' | T>C | 16362 (HV1) |
| 16390A (SEQ ID NO.:66) | 5'-TCA GAT AGG ACT CCC TTG -3' | G>A | 16390 (HV1) |
| 73G (SEQ ID NO.:67) | 5'-CTG GGG GGT GTG CAC GCG A -3' | A>G | 73 (HV2) |
| 93G (SEQ ID NO.:68) | 5'-ATA GCA TTG CGG GAC GCT G -3' | A>G | 93 (HV2) |
| 143A (SEQ ID NO.:69) | 5'-CTT TGA TTC CTA CCT CAT CCT A -3' | G>A | 143 (HV2) |
| 146C (SEQ ID NO.:70) | 5'-CTT TGA TTC CTG CCC CAT -3' | T>C | 146 (HV2) |
| 150T (SEQ ID NO.:71) | 5'-TCC TGC CTC ATT CTA TTA T -3' | C>T | 150 (HV2) |
| 152C (SEQ ID NO.:72) | 5'-CAT CCC ATT ATT TAT CGC -3' | T>C | 152 (HV2) |
| 153G (SEQ ID NO.:73) | 5'-CAT CCT GTT ATT TAT CGC -3' | A>G | 153 (HV2) |
| 153C (SEQ ID NO.:74) | 5'-CAT CCT CTT ATT TAT CGC -3' | A>C | 153 (HV2) |
| 189G (SEQ ID NO.:75) | 5'-TAT TAC AGG CGA GCA TAC -3' | A>G | 189 (HV2) |
| 195C (SEQ ID NO.:76) | 5'-GCG AAC ATA CCT ACT AAA GTG -3' | T>C | 195 (HV2) |
| 199C (SEQ ID NO.:77) | 5'-GAA CAT ACT TAC CAA AGT GTG TTA ATT -3' | T>C | 199 (HV2) |
| 200G (SEQ ID NO.:78) | 5'-CAT ACT TAC TGA AGT GTG TTA -3' | A>G | 200 (HV2) |
| 204C (SEQ ID NO.:79) | 5'-CAT ACT TAC TAA AGC GTG T -3' | T>C | 204 (HV2) |
| 207A (SEQ ID NO.:80) | 5'-ACT AAA GTG TAT TAA TTA ATT -3' | G>A | 207 (HV2) |
| 209C (SEQ ID NO.: 81) | 5'-GTG TCA ATT AAT TAA TGC -3' | T>C | 209 (HV2) |
| 210G (SEQ ID NO.: 82) | 5'-AAA GTG TGT TGA TTA ATT AAT -3' | A>G | 210 (HV2) |
| 214G (SEQ ID NO.: 83) | 5'-TGT GTT AAT TGA TTA ATG CTT -3' | A>G | 214 (HV2) |
| 235G (SEQ ID NO.:84) | 5'-TTG TAG GAC ATA GTA ATA ACA ATT GA -3' | A>G | 235 (HV2) |
| 237G (SEQ ID NO.: 85) | 5'-AGG ACA TAA TGA TAA CAA T -3' | A>G | 237 (HV2) |
| 249A (SEQ ID NO.:86) | 5'-ATA ACA ATT GAA TGT CTG C -3' | | 249 (HV2) |
| 257G (SEQ ID NO.:87) | 5'-TGT CTG CGC AGC CGC T -3' | A>G | 257 (HV2) |
| 263A (SEQ ID NO.:88) | 5'-CAG CCA CTT TCC ACA CAG -3' | G>A | 263 (HV2) |
| 281G (SEQ ID NO.: 89) | 5'-CAC AGA CAT CGT AAC AAA AAA -3' | A>G | 281 (HV2) |
| 281T (SEQ ID NO.:90) | 5'-CAC AGA CAT CTT AAC AAA AAA -3' | A>T | 281 (HV2) |

Serving to identify the mutation of human mtDNA, the DNA sequences of Table 1, useful in the present invention, aim to detect SNPs, or variations in base sequence or genes. These DNA sequences, which contain SNPs occurring in HV1 or HV2 genes of human mtDNA, account for variation in human mtDNA. In Table 1, the positions of these DNA sequences are also represented. Therefore, the polynucleotide probes according to the present invention preferably have base sequences complementary to the DNA sequences of SEQ ID NOS.: 1 to 90, so that hybridization therebetween can occur. For hybridization with the probes, either strand of the PCR products from the human mtDNA samples can be used. Thus, the probes according to the present invention may have base sequences identical to the DNA sequences of SEQ ID NOS.: 5 to 90 so that they react with the target DNA PCR products, the base sequences of which are complementary to the DNA sequences of SEQ ID NOS.: 5 to 90. In the present invention, base sequences complementary to the DNA sequences of the PCR products for the sequences of Table 1 are used as probes, and are named 16066G to 281T, respectively.

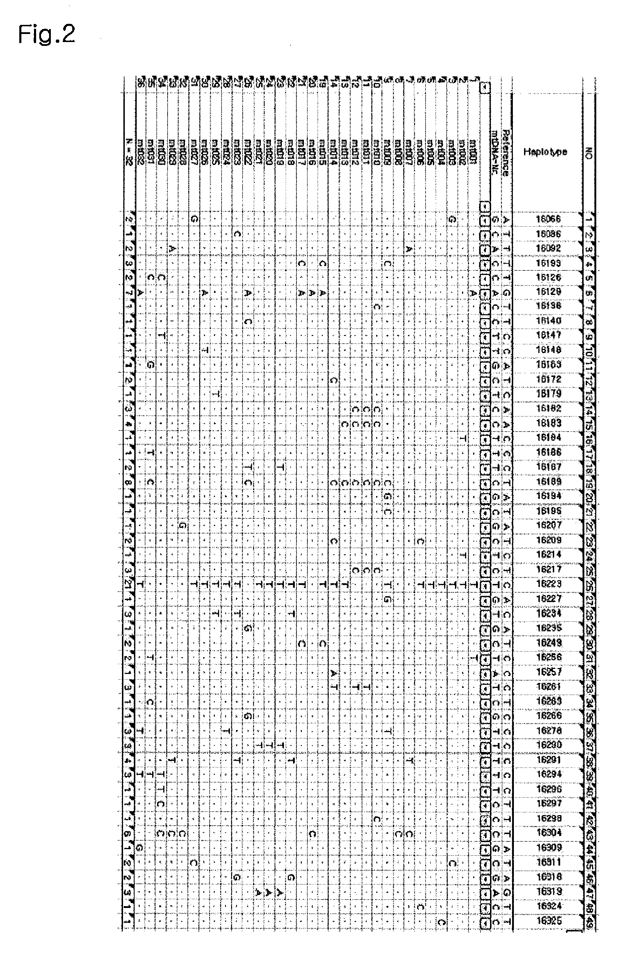

FIG. 2 is a base sequence information table showing SNP loci of polynucleotide probes in accordance with an embodiment of the present invention. As given in the information table, the polynucleotide probes having the base sequences of the sequence identification numbers of the present invention were constructed on the basis of analysis data from a large amount of human mtDNA. In greater detail, mtDNAs were isolated from 953 persons of varying age and gender and, of them, the mtDNAs which comprised the control region were analyzed for entire base sequence.

In FIG. 2, 49 SNP loci detected in 32 mtDNA sequences (mt001 ~ mt032) are summarized for the purpose of illustration. The term "SNP loci" as used herein refers to nucleotide positions at which nucleotides different from those of typical base sequences of human mtDNA exist. The portions expressed as ". (dot)" represent genotypes identical to those of general mtDNA sequences. In FIG. 2, for example, the nucleotide "16223" is the SNP locus at which base C is changed into base T.

The polynucleotide probe according to the present invention has one selected from among base sequences of SEQ ID NOS.: 5 to 90, which are also complementary to base sequences around SNP loci and thus can hybridize with base sequences having the SNP loci.

In FIG. 2, only base sequences corresponding to some of the SNP loci in HV 1 and HV2 of human mtDNA, that is, only base sequences corresponding to 49 SNP loci, are summarized for the purpose of illustration. In other words, the present invention is not limited to the base sequences of FIG. 2, but the probes according to the present invention comprise all of the base sequences represented by SEQ ID NOS.: 5 to 90 of Table 1. Not all of the mtDNA sequences of 953 persons, each 16,569 bp long, can be expressed because of limited space. On the basis of the same data analysis for other loci as in FIG. 2, probes having the base sequences of SEQ ID NOS.: 5 to 90 were constructed.

Leading to the present invention, intensive and thorough research into the identification of mutations of human mtDNA, conducted by the present inventors, resulted in the finding that a DNA sequence corresponding to one selected from among the base sequences of SEQ ID NOS.: 5 to 90, derived from HV1 and HV2 genes of human mtDNA, is optimal for the identification of mutations of human mtDNA. Also, it was found that base sequences identical or complementary to SEQ ID NOS.: 5 to 90 can be used as probes capable of identifying genetic mutations more accurately than conventional methods. Constructed on the basis of SNP loci of human mtDNA from a great number of persons, the polynucleotide probes of the present invention hybridize with mutant DNAs of interest, but not with other mutants.

As used herein, the term "polymorphism" refers to the occurrence of two or more substitution sequences or alleles in a genetically defined population. A polymorphic marker is the locus at which polymorphism occurs. A preferable polymorphic marker has two or more alleles having a frequency of 1 % or higher, preferably 10% or higher, and more preferably 20% or higher. The polymorphic locus may be a single base pair.

Therefore, nucleotide sequences, nucleic acid fragments comprising the same or complements thereof, as well as the above-described base sequences, are within the scope of the present invention. In consideration of limitative mutations occurring in one individual human DNA sample and the difference in origins of humans according to race and nation, a more preferable form of the polymorphic locus according to the present invention may be expressed as the same genotype from two or more individuals. The polymorphic loci according to the present invention is the occurrence of two or more substitutive sequences or alleles in one genetically defined group. Base sequences comprising DNA sequences identical to or complementary to the base sequence given in FIG. 2 may be employed.

As long as it comprises a base sequence which can complementarily bind to one of the DNA sequences of SEQ ID NOS.: 5 to 90, any polynucleotide probe based on the polymorphic loci may be used in the present invention. A continuous polynucleotide sequence, which comprises a base sequence able to complementarily bind to one of the DNA sequences of SEQ ID NOS.: 5 to 90 or a complement thereof, preferably ranges in length from 10 to 100 nucleotides, more preferably from 10 to 50 nucleotides, and most preferably from 14 to 30 nucleotides as shown in FIG. 2.

Targeting the SNP loci of the HV1 or HV2 gene of human mtDNA, the polynucleotide probes hybridize with the isolated human DNA of interest or the PCR products from the DNA. This hybridization differs according to the mutations of human mtDNA. In the present invention, thus, hybridization between the probes and the target is the criterion for identifying the mutation of human mtDNA. The kinds of mutations of mtDNA that can be analyzed using the probes of the present invention are as described in the sequence mutations and mutation loci of Table 1.

The sequence mutation in Table 1 refers to an SNP that is different from typical base sequences. For example, as for the probe "16066G", it has G at position 16066 of mtDNA, while a typical one has A at the same position. The base sequence of SEQ ID NO.: 5 according to the present invention can specifically bind to this mutation. By the term "hybridization depending on the mutation of human mtDNA", it is meant that the polynucleotide probe of SEQ ID NO.: 5 (a polynucleotide probe comprising a base sequence complementary to the DNA sequence of SEQ ID NO.: 5, hereinafter also true of DNA sequences of other SEQ. ID. NOS) hybridizes specifically with target DNA having "G" at position 16066 of human mtDNA or target PCR products.

It is preferable that the polynucleotide probes of SEQ ID NOS.: 5 to 90 bind to base sequences containing SNPs at positions 16066, 16075, 16086, 16093, 161.11, 16124, 16126, 16129, 16126 and 16129, 16136, 16140, 16154, 16162, 16172, 16174, 16192, 16209, 16213, 16217, 16223, 16217 and 16223, 16224, 16227, 16231, 16232, 16234, 16235, 16243, 16245, 16249, 16256, 16257, 16260, 16261, 16257 and 16261, 16265, 16266, 16270, 16274, 16278, 16264 and 16270 and 16278, 16284, 16288, 16290, 16291, 16293, 16294, 16295, 16297, 16298, 16304, 16309, 16311, 16316, 16319, 16324, 16325, 16327, 16355, 16357, 16362, 16390, 73, 93, 143, 146, 150, 152, 153, 153, 189, 195, 199, 200, 204, 207, 209, 210, 214, 235, 237,249,257,263,281, and 281 of human mtDNA.

The base residues of the SNPs at positions 16066 to 16140 are respectively G, C, C, C, T, C, C, A, C and A, C, and C. The base residues of the SNPs at positions 16162 to 237 are respectively, G, C, T, T, C, A, C, T, C and T, C, G, C, A, T, G, C, T, C, T, A, T, T, A and T, T, T, T, A, T, T and T and T, G, C, T, T, G, T, T, C, C, C, G, C, G, A, C, C, T, T, C, C, A, G, G, A, C, T, C, G, C, G, C, C, G, C, A, C, G, G, G, and G. More preferably, the base residues of the SNPs at positions 257 to 281 are respectively G, A, G, and T.

As described above, the present invention uses one of the DNA sequences of SEQ ID NOS.: 5 to 90 to hybridize with base sequences containing SNPs located in human mtDNA isolated from a great number of human individuals. That is, a polynucleotide probe comprising a base sequence identical to or complementary to one of the DNA sequences of SEQ ID NOS.: 5 to 90 is hybridized with human mtDNA isolates of interest (more accurately, the PCR product).

Constructed on the basis of SNP loci of human mtDNA, the polynucleotide probes of the present invention hybridize with mtDNAs having mutations of interest, but not with other mutants. In order to obtain more reliable data, the polynucleotide probes are preferably hybridized one or more times. Prior to the examination of hybridization, thus, hybridization between isolated DNA and a probe according to the present invention can be repeated to verify it. Subsequent to the examination of hybridization, in addition, hybridization between isolated DNA and other probes according to the present invention may be further conducted in a repeated manner in order to yield data based on various probes. It is preferable that the hybridization with the polynucleotide probe be repeated one or more times (particularly three or four times).

In accordance with another aspect thereof, the present invention provides a polynucleotide probe for use in identifying the mutation of human mtDNA, comprising a consecutive nucleotide sequence 14 - 30 bp long, identical or complementary to one of the DNA sequences of SEQ ID NOS.: 5 to 90. In accordance with a further aspect thereof, the present invention provides a DNA chip for use in identifying the mutation of human mtDNA, comprising an assembly of polynucleotide probes, each being a consecutive nucleotide sequence 14 - 30 bp long identical or complementary to one of the DNA sequence of SEQ ID NOS.: 5 to 90 and being capable of hybridizing with a base sequence containing an SNP locus on HV1 or HV2 of the control region of human mtDNA. In a preferable embodiment, the polynucleotide probes are immobilized on a glass substrate.

In the present invention, many human mtDNA mutations and haplotypes can be rapidly and accurately inspected using a DNA microarray technique. For example, about 20 probes for identifying the mutations of human mtDNA are integrated in a plurality of sections on one glass slide coated with chemically functional groups. Hybridization with a target gene sequence of interest can be examined with a fluorescent label produced through symmetric or asymmetric PCR.

As described above, the polynucleotide probes, each being a 15-30 bp long consecutive base sequence identical or complementary to one of the DNA sequences of SEQ ID NOS.: 5 to 90, are constructed on the basis of SNP loci of human mtDNA, so that they can hybridize with mtDNAs having mutations of interest, but not with other mutants. Therefore, the polynucleotide probes having base sequences identical or complementary to SEQ ID NOS.: 5 to 90 allow human mtDNA variation to be identified more efficiently than any other conventional method.

In accordance with still a further aspect thereof, the present invention provides a kit for use in identifying the mutation of human mtDNA.

The kit according to the present invention comprises: a polynucleotide probe, comprised of a consecutive nucleotide sequence 14 - 30 bp long identical or complementary to one of the DNA sequences of SEQ ID NOS.: 5 to 90, for hybridizing with a base sequence containing an SNP locus on HV1 or HV2 of the control region of human mtDNA; and a set of PCR primers for amplifying human mtDNA.

In combination with a microarray technique, the kit for use in identifying the mutation of human mtDNA in accordance with the present invention allows group-specific SNP to be determined according to hybridization with the polynucleotide probe, so that human mtDNA variations and haplotypes can be examined rapidly and accurately without base sequencing human mtDNA. In addition, DNA samples isolated from a plurality of human individuals can be analyzed for type of variation using the kit according to the present invention, which is applicable for the standardization and automation of the taxonomy of human mtDNA genotypes. Consequently, the present invention greatly reduces the time it takes to analyze samples using conventional methods, and has sufficient capacity to treat a large quantity of samples in a short period of time.

As elucidated above, the present invention can be used to identify human DNA samples having human mtDNA variations rapidly and accurately, thereby being applicable to various cases requiring the identification of individual persons.

For example, the present invention can be utilized in criminal investigation. That is, criminal suspect samples or forensic specimens can be analyzed for genotype or DNA variation using the method of the present invention, followed by the comparison of the gene pattern or DNA variation pattern between the suspect samples and the forensic specimens.

In accordance with still another aspect thereof, therefore, the present invention provides a method for identifying a forensic specimen, comprising: analyzing a sample from a suspect for mtDNA variation; analyzing a forensic specimen for mtDNA variation; and comparing the mtDNA variation between the suspect sample and the forensic specimen to determine whether the forensic specimen is derived from the suspect, wherein the human mtDNA variation is analyzed by isolating DNA from blood, cells or tissues of the suspect or the forensic specimen; amplifying the isolated DNA through PCR to produce a PCR product; hybridizing the PCR product with a polynucleotide probe comprising a base sequence identical or complementary to one of the DNA sequences of SEQ ID NOS.: 5 to 90; and examining the hybridization to determine variation in the mtDNA of the suspect or the forensic specimen.

Where the gene pattern or DNA variation pattern of the suspect is consistent with that of the forensic specimen within an effective range, it is possible to determine that the suspect is the criminal. As such, the present invention is applicable to various inspection methods based on human mtDNA variation patterns. It will also be apparent that these inspection methods fall within the scope of the present invention.

Furthermore, the present invention can be applied to search for missing children. Taking advantage of the fact that mitochondrial DNA is inherited through the maternal lineage, it is possible to determine maternity for a missing child through the analysis of mtDNA isolated from both. This is applicable to any person who maintains the same maternal lineage, even if she is not the mother of the missing child. In addition, since hundreds of mitochondria are present in one cell and their DNA is in a stable circular form, mtDNA variations can be used to identify corpses in mass incidents (e.g, airplane crash, building collapse, etc.).

A better understanding of the present invention may be obtained in light of the following examples, which are set forth to illustrate, but are not to be construed to limit the present invention.

### EXAMPLE 1: Preparation of Primers For Amplifying Isolated DNA

Primers for amplifying DNA isolated from humans were synthesized. Given in Table 2 are the primers that were used for symmetric or asymmetric PCR. Amplification conditions for PCR using the primers are summarized in Table 3, below.

**TABLE 2**

| Primers for Amplifying DNA for use in analysis of mtDNA Genotype | |
|---|---|
| Primers | Base Sequences |
| HVR1I sense (Sequence Information 1) | 5'- TTAACTCCACCATTAGCACC-3' |
| HVRI2 antisense (Sequence Information 2) | 5'-GAGGATGGTGGTCAAGGGAC-3' |
| HVRII3 sense (Sequence Information 3) | 5'-CACCCTATTAACCACTCACG-3' |
| HVRII antisense (Sequence Information 4) | 5'-TGAGATTAGTAGTATGGGAG-3' |

| | |
|---|---|
| 1. HVR: Hyper Variable Region 2. HVRI: nps16024-16365 3. HVRII: nps73-340 | |

**TABLE 3: PCR Conditions**

| Rxn Composition | Rxn Conditions | |
|---|---|---|
| Deionized Water 12.5ul | 94°C, 30 sec | 1 cycle |
| 10X buffer 2ul | 94°C, 15 sec | 36-38 cycles |
| 2mM dNTP 1ul | 56°C, 45 sec | |
| 10uM sense 1ul | 72°C, 30 sec | |
| 10uM antisense 1ul | 72°C, 5 min | 1 cycle |
| 1unit Taq 0.5ul | | |
| Pure DNA 2ul | | |

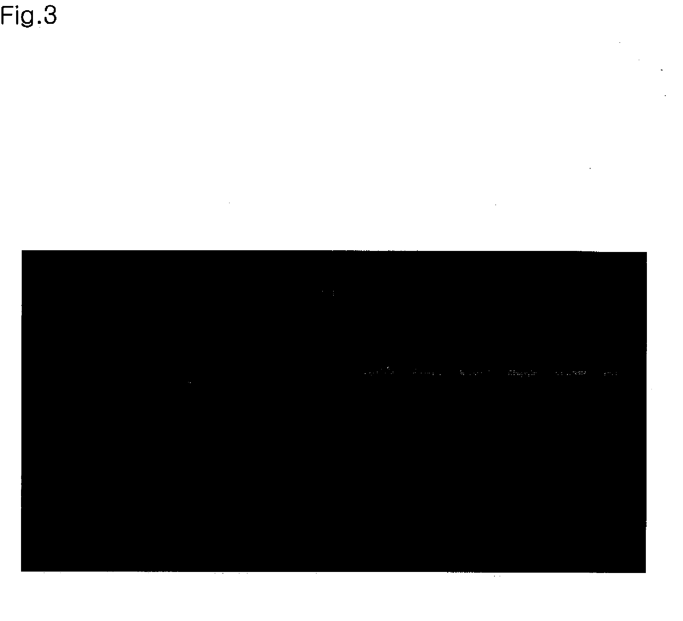

For fluorescence, the reverse (antisense) primers for symmetric or asymmetric PCR were designed to have rhodamine, cy3 or cy5, or biotin at the terminus thereof. After hybridization, the primers were associated with Streptavidin-Cyanine. The primers used in the present invention were synthesized by Metabion, Germany.
1) Human mtDNA was isolated using a DNeasy tissue kit, commercially available from Qiagen.
2) Symmetric PCR was performed in a DNA Engine (MJ Research, USA) under the conditions given in Table 3.
3) Asymmetric PCR was performed in a DNA Engine (MJ Research, USA) under the conditions given in Table 3.
4) A mixture of 5µl of the PCR product and 1 µl of gel loading buffer (0.2% orange G, 0.25% xylene cyanol FF, 60% glycerol) was electrophoresed on 2% agarose gel containing 1 µg/ml of bromide, followed by visualization of PCR bands in an image analyzer (Hitachi, Japan) equipped with a UV transilluminator (refer to FIG. 3).

FIG. 3 is a photograph showing PCR products of human mtDNA, in which HV1 regions amplified from genetically non-related human samples are electrophoresed in lanes 1 to 6 and HV2 regions amplified from the same human sample are electrophoresed in lanes 8-12 lanes, along with a PHIX174 DNA/BsuRI(HaeIII) size marker on lane 7.

### EXAMPLE 2: Synthesis of Probes According to Mutations and Immobilization thereof

Probes were synthesized for identifying human mtDNA mutations. The sequence numbers and base sequences of the probes for hybridization are listed in Table 1.

For the construction of a DNA chip, the probes according to the present invention were immobilized. First, an amino linker was attached to the 5' end of the probes. In order to prevent the spatial interference between the probes integrated on a glass slide upon hybridization, A 10-20 oligo-dT was also added to each of the probes. The probes were immobilized on the glass slide through a covalent bond between the amino group and the aldehyde group attached on the glass slide. The synthesis of the probes was conducted by Metabion, Germany.

### EXAMPLE 3: Construction of DNA Chip

1) In order to construct oligonucleotide chips, probes linked with an amino linker at a concentration of 50 pmole were mixed with one volume of 3X SSC and integrated on CSS-100 silylated slides (Cell Associate, U.S.A.), followed by incubation at room temperature for 16 hours. After completion of the reaction, the slides were washed twice with 0.2% SDS for 5 min and twice with distilled water for 5 min.
2) The slides were reacted with sodium borohydride (1.3g NaBH4 375ml PBS, 125m1 100% EtOH) for 5 min, incubated in boiling distilled water for 3 min and dried through centrifugation at 800 rpm for 10 min.
3) For a plurality of sample reactions on one slide, separation was conducted with a perfusion chamber (BioGrace, U.S.A.), and the slides were stored at room temperature in a dark place.

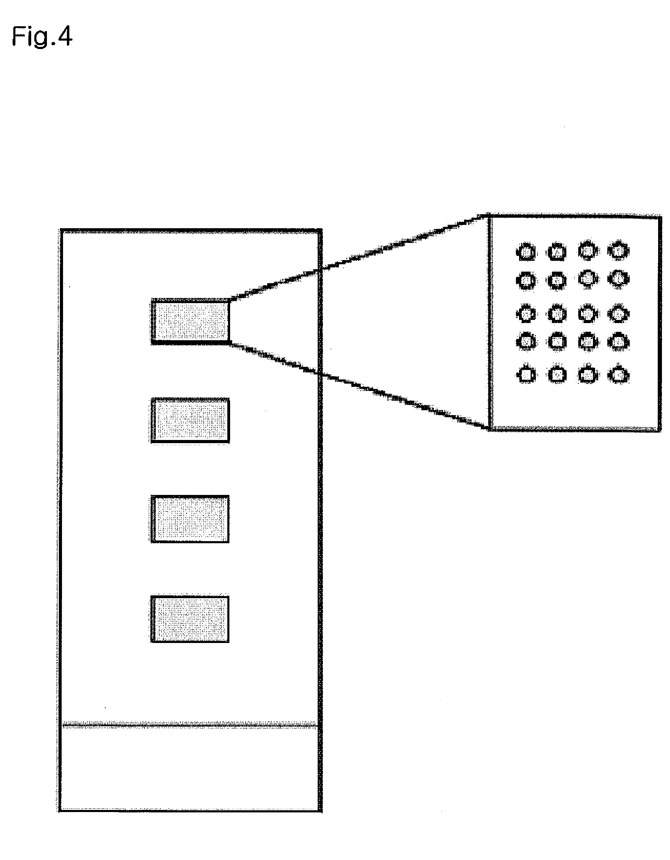

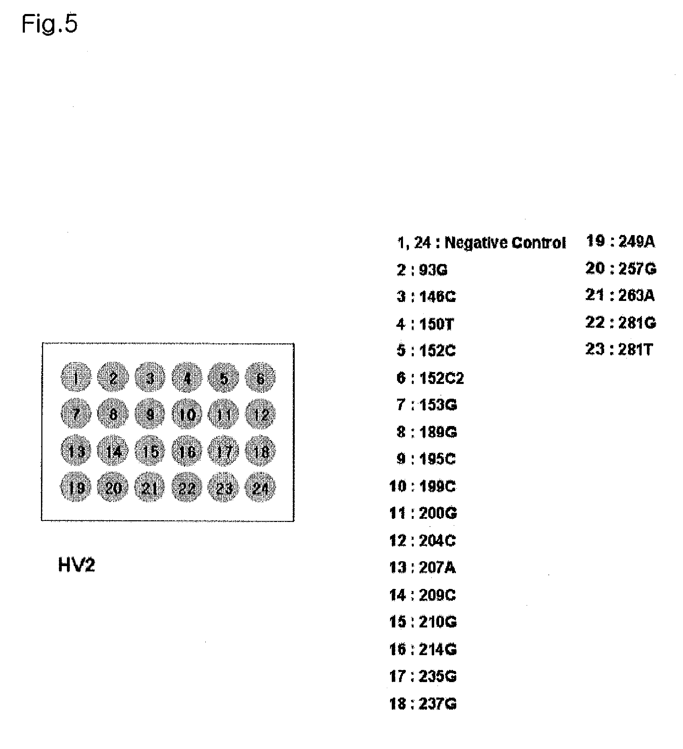

The DNA chip thus constructed is illustrated in FIGS. 4 and 5. FIG. 4 is a schematic diagram showing the structure of a DNA chip containing polynucleotide probes according to a preferable embodiment of the present invention. FIG. 5 is a schematic diagram showing the constitution of a DNA chip in which polynucleotide probes responsible for the HV2 gene are arranged. In the DNA chip of FIG. 5, an arrangement of 24 probes is illustrated, along with the names thereof.

### EXAMPLE 4: Hybridization

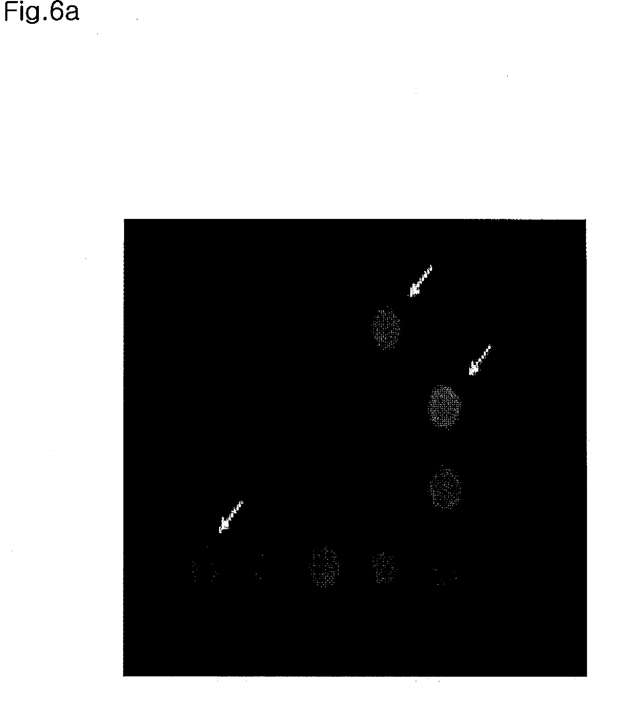

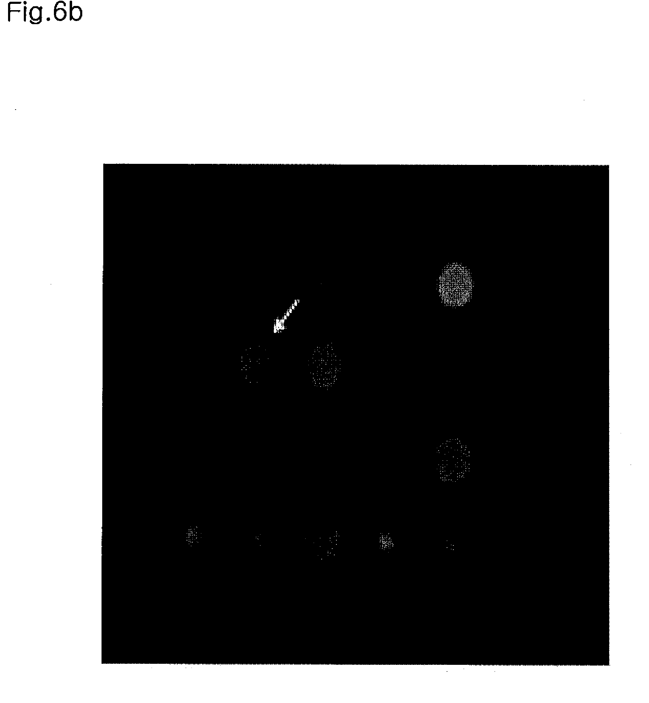

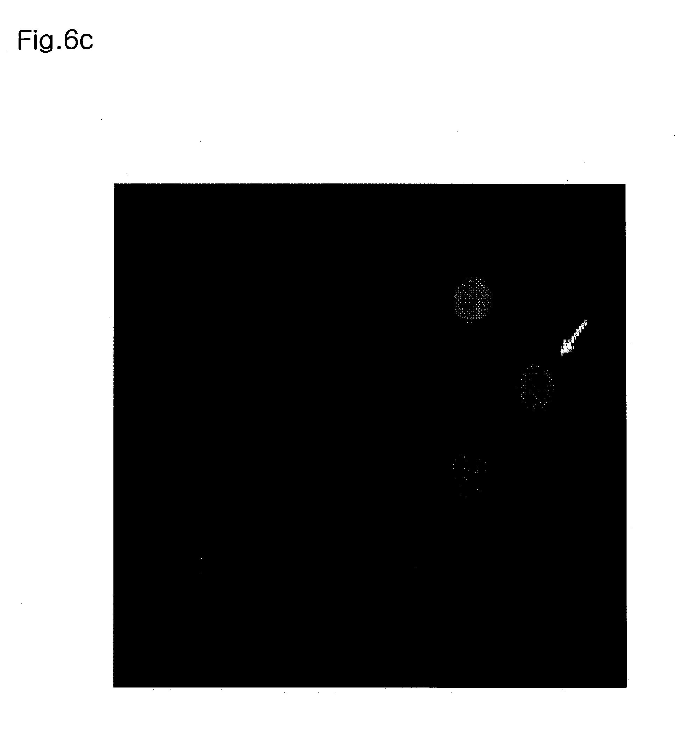

1) The rhodamine-, cy3-, cy5- or biotin-associated PCT product was mixed with a hybridization buffer (3X SSC, 0.25% SDS) at a volume ratio of 1:9. When biotin was used for fluorescent reaction, Streptavidin-Cyanine was added at a concentration of 1 µg/ml.
2) The mixture in the hybridization buffer was applied to the chamber, followed by incubation at 60°C for 1 hour.
3) The slides were first washed with 1X SSC, 0.1%SDS for 5 min and then with O.1X SSC for 3 min.
4) The washed slides were dried through centrifugation at 800 rpm for 10 min.
5) Using a LuxScan-10K/A scanner (CapitalBio, China), the samples were measured for fluorescence to analyze the genotypes thereof. The results are given in FIGS. 6A to 6C.

FIGS. 6A to 6C are photographs showing results from the hybridization of PCR products of human mtDNA with the polynucleotide probes of the present invention, that is, probes 150T, 200G and 249A (A), probe 189G (B) and probe 204C (C). As seen in these figures, the fluorescence detected is different form one sample to another sample, which indicates that the present invention can be applied to the identification of mtDNA isolates of interest.

The present invention has been described in an illustrative manner, and it is to be understood that the terminology used is intended to be in the nature of description rather than of limitation. Many modifications and variations of the present invention are possible in light of the above teachings. Therefore, it is to be understood that, within the scope of the appended claims, the invention may be practiced other than as specifically described.

As described hitherto, a method is provided for identifying the mutation of human mtDNA, comprising: isolating DNA from human blood, cells or tissues; amplifying the isolated DNA through PCR to produce a PCR product; hybridizing the PCR product with a polynucleotide probe containing a base sequence identical or complementary to one selected from a group consisting of nucleotide sequences of SEQ ID NOS.: 5 to 90; and examining the hybridization to determine human mtDNA variation.

According to the present invention, a plurality of mutations of human mtDNA and haplotypes of human mtDNA can be rapidly and accurately inspected on one slide. Featuring the use of the control region of human mtDNA as a target, the present invention can analyze human mtDNA genotypes and haplotypes far more rapidly and effectively than can conventional methods.

On the basis of the single nucleotide polymorphism (SNP) loci in the control region of human mtDNA, discovered by the present inventors, polynucleotide probes capable of specifically binding to gene variants of individuals were constructed, and thus can hybridize with mutant DNAs of interest, but not with other mutants.

Intensive and thorough research into the identification of mutation of human mtDNA, conducted by the present inventors, resulted in the finding that a DNA sequence corresponding to one selected from among the base sequences of SEQ ID NOS.: 5 to 90, derived from HV1 and HV2 genes of human mtDNA, is optimal for the identification of mutations of human mtDNA. Also, it was found that base sequences identical or complementary to SEQ ID NOS.: 5 to 90 can be used as probes capable of identifying genetic mutations far more accurately and effectively than conventional methods.

In combination with a microarray technique, the polynucleotide probes according to the present invention are used to determine SNP as a result of hybridization therewith, so that human mtDNA variations and haplotypes can be examined rapidly and accurately without base sequencing human mtDNA. In addition, DNA samples isolated from a plurality of human individuals can be analyzed for genotype, which is applicable for the standardization and automation of the taxonomy of human mtDNA genotypes. Consequently, the present invention greatly reduces the time it takes to analyze samples with conventional methods and has the capacity to treat a quantity of samples within a short period of time.

## Claims

1. A method of identifying a mutation of human mitochondrial DNA, comprising the steps of:
isolating DNA from human blood, cells or tissues;
subjecting the isolated DNA to polymerase chain reaction (PCR) to produce a PCR product;
hybridizing the PCR product with a polynucleotide probe comprising a base sequence identical or complementary to one of DNA sequences of SEQ ID NOS.: 5 to 90; and
examining hybridization between the PCR product and the polynucleotide probe to determine the mutation of the human mitochondrial DNA.

2. The method as set forth in claim 1, wherein the PCR product comprises either or both of an HV1 region and an HV2 region located in a control region of the human mitochondrial DNA.

3. The method as set forth in claim 1, wherein the polynucleotide probe is a base sequence consisting of 14 ~ 30 consecutive nucleotide residues.

4. The method as set forth in claim 1, wherein the polynucleotide probe binds to a base sequence containing a single nucleotide polymorphism (SNP) at position 16066, 16075, 16086, 16093, 16111, 16124, 16126, 16129, 16126 and 16129, 16136, 16140, 16154, 16162, 16172, 16174, 16192, 16209, 16213, 16217, 16223, 16217 and 16223, 16224, 16227, 16231, 16232, 16234, 16235, 16243, 16245, 16249, 16256, 16257, 16260, 16261, 16257 and 16261, 16265, 16266, 16270, 16274, 16278, 16264 and 16270 and 16278, 16284, 16288, 16290, 16291, 16293, 16294, 16295, 16297, 16298, 16304, 16309, 16311, 16316, 16319, 16324, 16325, 16327, 16355, 16357, 16362, 16390, 73, 93, 143, 146, 150, 152, 153, 153, 189, 195, 199, 200, 204,207,209,210,214,235,237,249,257,263,281, or 281 of the human mitochondrial DNA.

5. The method as set forth in claim 4, wherein base residues of the SNP at positions from 16066 to 16140 are respectively G, C, C, C, T, C, C, A, C and A, C, and C, base residues of the SNP at positions from 16162 to 237 are respectively G, C, T, T, C, A, C, T, C and T, C, G, C, A, T, G, C, T, C, T, A, T, T, A and T, T, T, T, A, T, T and T and T, G, C, T, T, G, T, T, C, C, C, G, C, G, A, C, C, T, T, C, C, A, G, G, A, C, T, C, G, C, G, C, C, G, C, A, C, G, G, G, and G, or base residues of the SNP at positions from 257 to 281 are respectively G, A, G, and T.

6. The method as set forth in claim 1, wherein the hybridizing step is conducted one or more times.

7. Use of a polynucleotide probe for identifying a mutation of human mitochondrial DNA, comprising a base sequence consisting of 14 ~ 30 consecutive nucleotide residues, which is identical or complementary to one of DNA sequences ofSEQ ID NOS.: 5 to 90.

8. A DNA chip for identifying a mutation of human mitochondrial DNA, comprising a polynucleotide probe, which is a 14 - 30 consecutive bp long nucleotide sequence identical or complementary to one of the DNA sequences of SEQ ID NOS.: 5 to 90, and is capable of hybridizing with a base sequence containing an SNP locus on HV1 or HV2 of a control region of human mitochondrial DNA.

9. A kit for identifying a mutation of human mitochondrial DNA, comprising:
a polynucleotide probe, comprised of a consecutive nucleotide sequence 14 - 30 bp long identical or complementary to one of the DNA sequences of SEQ ID NOS.: 5 to 90, for hybridizing with a base sequence containing an SNP locus on HV1 or HV2 of a control region of human mitochondrial DNA; and
a set of primers for amplifying human mitochondrial DNA through PCR.

10. A method for identifying a forensic specimen, comprising:
analyzing a sample from a suspect for mitochondrial DNA variation;
analyzing a forensic specimen for mitochondrial DNA variation; and
comparing the mitochondrial DNA variation between the suspect sample and the forensic specimen to determine whether the forensic specimen is derived from the suspect,
wherein, the human mitochondrial DNA variations are analyzed by:
isolating DNA from blood, cells or tissues of the suspect or the forensic specimen;
amplifying the isolated DNA through PCR to produce a PCR product;
hybridizing the PCR product with a polynucleotide probe comprising a base sequence identical or complementary to one of the DNA sequences of SEQ ID NOS.: 5 to 90; and
examining the hybridization between the PCR product and the polynucleotide probe to determine variation of the mitochondrial DNA of the suspect or the forensic specimen.


<110>	Genocheck

<120>	Method, Polynucleotide Probe, DNA Chip and Kit for Identifying
	Mutation of Human Mitochondrial DNA

<130>	0001

<160>	90

<170>	KopatentIn 1.71

<210>	1
<211>	20
<212>	DNA
<213>	Artificial Sequence

<220>
<223>	primer for human mitochondria DNA

<400>	1
ttaactccac cattagcacc                                                    20

<210>	2
<211>	20
<212>	DNA
<213>	Artificial Sequence

<220>
<223>	primer for human mitochondria DNA

<400>	2
gaggatggtg gtcaagggac                                                    20

<210>	3
<211>	20
<212>	DNA
<213>	Artificial Sequence

<220>
<223>	primer for human mitochondria DNA

<400>	3
caccctatta accactcacg                                                    20

<210>	4
<211>	20
<212>	DNA
<213>	Artificial Sequence

<220>
<223>	primer for human mitochondria DNA

<400>	4
tgagattagt agtatgggag                                                    20

<210>	5
<211>	20
<212>	DNA
<213>	Artificial Sequence

<220>
<223>    probe for human mitochondria DNA

<400>    5
caagtattgg ctcacccatc                                                    20

<210>    6
<211>    17
<212>    DNA
<213>    Artificial Sequence

<220>
<223>    probe for human mitochondria DNA

<400>    6
ctcacccacc aacaacc                                                       17

<210>    7
<211>    21
<212>    DNA
<213>    Artificial Sequence

<220>
<223>    probe for human mitochondria DNA

<400>    7
caacaaccgc catgtatttc g                                                  21

<210>    8
<211>    21
<212>    DNA
<213>    Artificial Sequence

<220>
<223>    probe for human mitochondria DNA

<400>    8
cgctatgtat ctcgtacatt a                                                  21

<210>    9
<211>    21
<212>    DNA
<213>    Artificial Sequence

<220>
<223>    probe for human mitochondria DNA

<400>    9
ttactgccag tcaccatgaa t                                                  21

<210>    10
<211>    21
<212>    DNA
<213>    Artificial Sequence

<220>
<223>    probe for human mitochondria DNA

<400>    10
ccatgaatat cgtacggtac c                                                21

<210>    11
<211>    20
<212>    DNA
<213>    Artificial Sequence

<220>
<223>    probe for human mitochondria DNA

<400>    11
atgaatattg cacggtacca                                                  20

<210>    12
<211>    20
<212>    DNA
<213>    Artificial Sequence

<220>
<223>    probe for human mitochondria DNA

<400>    12
atgaatattg tacagtacca                                                  20

<210>    13
<211>    26
<212>    DNA
<213>    Artificial Sequence

<220>
<223>    probe for human mitochondria DNA

<400>    13
ccatgaatat tgcacagtac cataaa                                           26

<210>    14
<211>    20
<212>    DNA
<213>    Artificial Sequence

<220>
<223>    probe for human mitochondria DNA

<400>    14
gtaccacaaa tacttgacca                                                  20

<210>    15
<211>    20
<212>    DNA
<213>    Artificial Sequence

<220>
<223>    probe for human mitochondria DNA

<400>    15
gtaccataaa cacttgacca                                                  20

<210>	16
<211>	22
<212>	DNA
<213>	Artificial Sequence

<220>
<223>	probe for human mitochondria DNA


<400>	16
cttgaccacc tgtagtacat aa	22


<210>	17
<211>	21
<212>	DNA
<213>	Artificial Sequence

<220>
<223>	probe for human mitochondria DNA


<400>	17
ctgtagtaca tgaaaaccca a	21


<210>	18
<211>	19
<212>	DNA
<213>	Artificial Sequence

<220>
<223>	probe for human mitochondria DNA


<400>	18
aaaacccaac ccacatcaa	19


<210>	19
<211>	25
<212>	DNA
<213>	Artificial Sequence

<220>
<223>	probe for human mitochondria DNA


<400>	19
gtacataaaa acccaatcta catca	25


<210>	20
<211>	20
<212>	DNA
<213>	Artificial Sequence

<220>
<223>	probe for human mitochondria DNA


<400>	20
tcaaaacccc ctcctcatgc	20


<210>	21

<211>    20
<212>    DNA
<213>    Artificial Sequence

<220>
<223>    probe for human mitochondria DNA


<400>    21
tacaagcaag cacagcaatc                                                    20


<210>    22
<211>    22
<212>    DNA
<213>    Artificial Sequence

<220>
<223>    probe for human mitochondria DNA


<400>    22
caagcaagta caacaatcaa cc                                                 22


<210>    23
<211>    19
<212>    DNA
<213>    Artificial Sequence

<220>
<223>    probe for human mitochondria DNA


<400>    23
gcaagtacag caaccaacc                                                     19


<210>    24
<211>    24
<212>    DNA
<213>    Artificial Sequence

<220>
<223>    probe for human mitochondria DNA


<400>    24
cagcaatcaa ccttcaacta tcac                                               24


<210>    25
<211>    18
<212>    DNA
<213>    Artificial Sequence

<220>
<223>    probe for human mitochondria DNA


<400>    25
gcaaccaacc ctcaacta                                                      18


<210>    26
<211>    19
<212>    DNA
<213>    Artificial Sequence

<220>
<223>    probe for human mitochondria DNA

<400>    26
aatcaacccc caactatca                                                    19

<210>    27
<211>    20
<212>    DNA
<213>    Artificial Sequence

<220>
<223>    probe for human mitochondria DNA

<400>    27
caaccctcag ctatcacaca                                                   20

<210>    28
<211>    21
<212>    DNA
<213>    Artificial Sequence

<220>
<223>    probe for human mitochondria DNA

<400>    28
tcaactacca cacatcaact g                                                 21

<210>    29
<211>    22
<212>    DNA
<213>    Artificial Sequence

<220>
<223>    probe for human mitochondria DNA

<400>    29
cccctaacta taacacatca ac                                                22

<210>    30
<211>    21
<212>    DNA
<213>    Artificial Sequence

<220>
<223>    probe for human mitochondria DNA

<400>    30
tcaactatca tacatcaact g                                                 21

<210>    31
<211>    20
<212>    DNA
<213>    Artificial Sequence

<220>
<223>    probe for human mitochondria DNA

<400>    31
caactatcac gcatcaactg                                                    20

<210>    32
<211>    17
<212>    DNA
<213>    Artificial Sequence

<220>
<223>    probe for human mitochondria DNA

<400>    32
acatcaaccg caactcc                                                       17

<210>    33
<211>    21
<212>    DNA
<213>    Artificial Sequence

<220>
<223>    probe for human mitochondria DNA

<400>    33
acatcaactg taactccaaa g                                                  21

<210>    34
<211>    20
<212>    DNA
<213>    Artificial Sequence

<220>
<223>    probe for human mitochondria DNA

<400>    34
catcaactgc aaccccaaag                                                    20

<210>    35
<211>    17
<212>    DNA
<213>    Artificial Sequence

<220>
<223>    probe for human mitochondria DNA

<400>    35
ctccaaagtc acccctc                                                       17

<210>    36
<211>    18
<212>    DNA
<213>    Artificial Sequence

<220>
<223>    probe for human mitochondria DNA

<400>    36

tgcaactcca aagcaacc 18

<210> 37
<211> 17
<212> DNA
<213> Artificial Sequence

<220>
<223> probe for human mitochondria DNA

<400> 37
aaagccactc ctcaccc 17

<210> 38
<211> 15
<212> DNA
<213> Artificial Sequence

<220>
<223> probe for human mitochondria DNA

<400> 38
agccacctct caccc 15

<210> 39
<211> 18
<212> DNA
<213> Artificial Sequence

<220>
<223> probe for human mitochondria DNA

<400> 39
ccaaagcaac ctctcacc 18

<210> 40
<211> 14
<212> DNA
<213> Artificial Sequence

<220>
<223> probe for human mitochondria DNA

<400> 40
acccctcccc cact 14

<210> 41
<211> 17
<212> DNA
<213> Artificial Sequence

<220>
<223> probe for human mitochondria DNA

<400> 41
acccctcatc cactagg 17

<210> 42
<211> 19
<212> DNA
<213> Artificial Sequence

<220>
<223> probe for human mitochondria DNA


<400> 42
cctcacccat taggatacc 19


<210> 43
<211> 21
<212> DNA
<213> Artificial Sequence

<220>
<223> probe for human mitochondria DNA


<400> 43
cacccactag aataccaaca a 21


<210> 44
<211> 21
<212> DNA
<213> Artificial Sequence

<220>
<223> probe for human mitochondria DNA


<400> 44
cactaggata tcaacaaacc t 21


<210> 45
<211> 23
<212> DNA
<213> Artificial Sequence

<220>
<223> probe for human mitochondria DNA


<400> 45
cccttaccca ttaggatatc aac 23


<210> 46
<211> 19
<212> DNA
<213> Artificial Sequence

<220>
<223> probe for human mitochondria DNA


<400> 46
ataccaacag acctaccca 19


<210> 47
<211> 19
<212> DNA

<213>    Artificial Sequence

<220>
<223>    probe for human mitochondria DNA


<400>    47
   caacaaaccc acccaccct                                                    19


<210>    48
<211>    20
<212>    DNA
<213>    Artificial Sequence

<220>
<223>    probe for human mitochondria DNA


<400>    48
aacctatcca cccttaacag                                                    20


<210>    49
<211>    20
<212>    DNA
<213>    Artificial Sequence

<220>
<223>    probe for human mitochondria DNA


<400>    49
aacctactca cccttaacag                                                    20


<210>    50
<211>    18
<212>    DNA
<213>    Artificial Sequence

<220>
<223>    probe for human mitochondria DNA


<400>    50
aacctacccg cccttaac                                                      18


<210>    51
<211>    20
<212>    DNA
<213>    Artificial Sequence

<220>
<223>    probe for human mitochondria DNA


<400>    51
aacctaccca tccttaacag                                                    20


<210>    52
<211>    22
<212>    DNA
<213>    Artificial Sequence

<220>

<223>    probe for human mitochondria DNA

<400>    52
aacctaccca ctcttaacag ta                                                22

<210>    53
<211>    19
<212>    DNA
<213>    Artificial Sequence

<220>
<223>    probe for human mitochondria DNA

<400>    53
tacccacccc taacagtac                                                    19

<210>    54
<211>    20
<212>    DNA
<213>    Artificial Sequence

<220>
<223>    probe for human mitochondria DNA

<400>    54
acccaccctc aacagtacat                                                   20

<210>    55
<211>    20
<212>    DNA
<213>    Artificial Sequence

<220>
<223>    probe for human mitochondria DNA

<400>    55
taacagcaca tagtacataa                                                   20

<210>    56
<211>    20
<212>    DNA
<213>    Artificial Sequence

<220>
<223>    probe for human mitochondria DNA

<400>    56
cttaacagta catggtacat                                                   20

<210>    57
<211>    21
<212>    DNA
<213>    Artificial Sequence

<220>
<223>    probe for human mitochondria DNA

<400>    57
cagtacatag cacataaagc c                                                21

<210>    58
<211>    21
<212>    DNA
<213>    Artificial Sequence

<220>
<223>    probe for human mitochondria DNA

<400>    58
atagtacatg aagccattta c                                                21

<210>    59
<211>    21
<212>    DNA
<213>    Artificial Sequence

<220>
<223>    probe for human mitochondria DNA

<400>    59
agtacataaa accatttacc g                                                21

<210>    60
<211>    21
<212>    DNA
<213>    Artificial Sequence

<220>
<223>    probe for human mitochondria DNA

<400>    60
ataaagccat ctaccgtaca t                                                21

<210>    61
<211>    21
<212>    DNA
<213>    Artificial Sequence

<220>
<223>    probe for human mitochondria DNA

<400>    61
ataaagccat tcaccgtaca t                                                21

<210>    62
<211>    21
<212>    DNA
<213>    Artificial Sequence

<220>
<223>    probe for human mitochondria DNA

<400>    62
aagccattta tcgtacatag c                                                21

<210> 63
<211> 19
<212> DNA
<213> Artificial Sequence

<220>
<223> probe for human mitochondria DNA

<400> 63
gtcaaatcct ttctcgccc 19

<210> 64
<211> 21
<212> DNA
<213> Artificial Sequence

<220>
<223> probe for human mitochondria DNA

<400> 64
tcaaatccct cctcgtcccc a 21

<210> 65
<211> 16
<212> DNA
<213> Artificial Sequence

<220>
<223> probe for human mitochondria DNA

<400> 65
ccttctcgcc cccatg 16

<210> 66
<211> 18
<212> DNA
<213> Artificial Sequence

<220>
<223> probe for human mitochondria DNA

<400> 66
tcagatagga gtcccttg 18

<210> 67
<211> 19
<212> DNA
<213> Artificial Sequence

<220>
<223> probe for human mitochondria DNA

<400> 67
   ctggggggtg tgcacgcga 19

<210> 68
<211> 19

<212>    DNA
<213>    Artificial Sequence

<220>
<223>    probe for human mitochondria DNA

<400>    68
atagcattgc gggacgctg                                                    19

<210>    69
<211>    22
<212>    DNA
<213>    Artificial Sequence

<220>
<223>    probe for human mitochondria DNA

<400>    69
ctttgattcc tacctcatcc ta                                                22

<210>    70
<211>    18
<212>    DNA
<213>    Artificial Sequence

<220>
<223>    probe for human mitochondria DNA

<400>    70
ctttgattcc tgccccat                                                     18

<210>    71
<211>    19
<212>    DNA
<213>    Artificial Sequence

<220>
<223>    probe for human mitochondria DNA

<400>    71
tcctgcctca ttctattat                                                    19

<210>    72
<211>    18
<212>    DNA
<213>    Artificial Sequence

<220>
<223>    probe for human mitochondria DNA

<400>    72
catcccatta tttatcgc                                                     18

<210>    73
<211>    18
<212>    DNA
<213>    Artificial Sequence

<220>
<223>    probe for human mitochondria DNA


<400>    73
catcctgtta tttatcgc                                                    18


<210>    74
<211>    18
<212>    DNA
<213>    Artificial Sequence

<220>
<223>    probe for human mitochondria DNA


<400>    74
catcctctta tttatcgc                                                    18


<210>    75
<211>    18
<212>    DNA
<213>    Artificial Sequence

<220>
<223>    probe for human mitochondria DNA


<400>    75
tattacaggc gagcatac                                                    18


<210>    76
<211>    21
<212>    DNA
<213>    Artificial Sequence

<220>
<223>    probe for human mitochondria DNA


<400>    76
gcgaacatac ctactaaagt g                                                21


<210>    77
<211>    27
<212>    DNA
<213>    Artificial Sequence

<220>
<223>    probe for human mitochondria DNA


<400>    77
gaacatactt accaaagtgt gttaatt                                          27


<210>    78
<211>    21
<212>    DNA
<213>    Artificial Sequence

<220>
<223>    probe for human mitochondria DNA

<400>    78
catacttact gaagtgtgtt a                                                21

<210>    79
<211>    19
<212>    DNA
<213>    Artificial Sequence

<220>
<223>    probe for human mitochondria DNA

<400>    79
catacttact aaagcgtgt                                                   19

<210>    80
<211>    21
<212>    DNA
<213>    Artificial Sequence

<220>
<223>    probe for human mitochondria DNA

<400>    80
actaaagtgt attaattaat t                                                21

<210>    81
<211>    18
<212>    DNA
<213>    Artificial Sequence

<220>
<223>    probe for human mitochondria DNA

<400>    81
gtgtcaatta attaatgc                                                    18

<210>    82
<211>    21
<212>    DNA
<213>    Artificial Sequence

<220>
<223>    probe for human mitochondria DNA

<400>    82
aaagtgtgtt gattaattaa t                                                21

<210>    83
<211>    21
<212>    DNA
<213>    Artificial Sequence

<220>
<223>    probe for human mitochondria DNA

<400>    83
tgtgttaatt gattaatgct t                                                21

<210> 84
<211> 26
<212> DNA
<213> Artificial Sequence

<220>
<223> probe for human mitochondria DNA


<400> 84
ttgtaggaca tagtaataac aattga 26


<210> 85
<211> 19
<212> DNA
<213> Artificial Sequence

<220>
<223> probe for human mitochondria DNA


<400> 85
aggacataat gataacaat 19


<210> 86
<211> 19
<212> DNA
<213> Artificial Sequence

<220>
<223> probe for human mitochondria DNA


<400> 86
ataacaattg aatgtctgc 19


<210> 87
<211> 16
<212> DNA
<213> Artificial Sequence

<220>
<223> probe for human mitochondria DNA


<400> 87
tgtctgcgca gccgct 16


<210> 88
<211> 18
<212> DNA
<213> Artificial Sequence

<220>
<223> probe for human mitochondria DNA


<400> 88
cagccacttt ccacacag 18


<210> 89

<211>    21
<212>    DNA
<213>    Artificial Sequence

<220>
<223>    probe for human mitochondria DNA


<400>    89
cacagacatc gtaacaaaaa a                                                21


<210>    90
<211>    21
<212>    DNA
<213>    Artificial Sequence

<220>
<223>    probe for human mitochondria DNA


<400>    90
cacagacatc ttaacaaaaa a                                                21